# EUROPÄISCHE PATENTSCHRIFT

(11) **EP 1 091 940 B1**
(45) Veröffentlichungstag und Bekanntmachung des Hinweises auf die Patenterteilung: **10.09.2003**
(21) Anmeldenummer: 99931126.9
(22) Anmeldetag: 21.06.1999
(51) Int. Cl.: C07D 231/00

(54) **VERFAHREN ZUR HERSTELLUNG VON 1-ALKYL-PYRAZOL-5-CARBONSÄUREESTERN**
METHOD FOR PREPARING 1-ALKYL-PYRAZOL-5-CARBOXYLIC ACID ESTERS
PROCEDE DE PREPARATION D'ESTERS D'ACIDE 1-ALKYLE-PYRAZOL-5-CARBOXYLIQUE

(30) Priorität: 02.07.1998 DE 19829616
(43) Veröffentlichungstag der Anmeldung: 18.04.2001
(73) Patentinhaber: Bayer Aktiengesellschaft, 51368 Leverkusen (DE)
(72) Erfinder: MÜLLER, Nikolaus, D-40789 Monheim (DE); MATZKE, Michael, D-42113 Wuppertal (DE)
(86) Internationale Anmeldenummer: EP9904297
(87) Internationale Veröffentlichungsnummer: WO00001673

(56) Entgegenhaltungen:
- EP-A- 0 854 142
- THEILHEIMER W: "SYNTHETIC METHODS OF ORGANIC CHEMISTRY, PASSAGE" SYNTHETIC METHODS OF ORGANIC CHEMISTRY, Bd. 2, 1975, Seite 128 XP002058628 THEILHEIMER W
- THEILHEIMER W: "SYNTHETIC METHODS OF ORGANIC CHEMISTRY" SYNTHETIC METHODS OF ORGANIC CHEMISTRY, Bd. 6, 1952, Seite 148 XP002058629 THEILHEIMER W
- DATABASE WPI Section Ch, Week 198615 Derwent Publications Ltd., London, GB; Class B03, AN 1986-096631 XP002121094 & JP 61 040266 A (MORISHITA PHARM CO LTD), 26. Februar 1986 (1986-02-26)
- DATABASE WPI Section Ch, Week 199531 Derwent Publications Ltd., London, GB; Class B03, AN 1995-234200 XP002121095 & JP 07 118238 A (UBE IND LTD), 9. Mai 1995 (1995-05-09)
- THEILHEIMER W: "SYNTHETIC METHODS OF ORGANIC CHEMISTRY. "PASSAGE TEXT"" SYNTHETIC METHODS OF ORGANIC CHEMISTRY, Bd. 16, 1962, Seite 204 XP002058626 THEILHEIMER W

## Beschreibung

Die vorliegende Erfindung betrifft ein Verfahren zur Herstellung von 1-Alkyl-, insbesondere auch 1,3-Dialkyl-pyrazol-5-carbonsäureestern, aus dem Enolat von 2,4-Dicarbonsäureestern und N-Alkylhydraziniumsalzen.

Es ist bereits bekannt, 1-Alkyl-pyrazol-5-carbonsäureester durch Alkylierung von Pyrazol-3-carbonsäureestern mit Alkylierungsmitteln (z.B. Alkylhalogeniden, Dialkylsulfaten oder Alkyltosylaten) herzustellen.

So beschreiben EP-OS 463 756 und EP-OS 526 004 die Herstellung von 1-Methyl-3-n-propyl-pyrazol-5-carbonsäureethylester durch die Umsetzung von 3-n-Propylpyrazol-5-carbonsäureethylester mit Dimethylsulfat. Offenbar entsteht hier ein Gemisch aus den beiden isomeren N-Methyl-pyrazolen, das aufwendig mittels chromatographischer Methoden aufgetrennt werden muß.

In der DE-OS 19 27 429 wird die Herstellung von 1-Methyl- und 1-Ethyl-3-propylpyrazol-5-carbonsäureethylester aus 3-Propyl-pyrazol-5-carbonsäureethylester durch Alkylierung mit Dimethylsulfat oder Triethyloxoniumtetrafluoroborat beschrieben. Es finden sich keine Angaben zum eventuellen Auftreten von Isomeren, ihrer Trennung oder der Gesamtausbeute des gewünschten Produktes, obwohl aufgrund der bis dahin bekannten Literatur immer mit der Entstehung von Isomerengemischen gerechnet werden muß. So führt die Methylierung von 3,4-Dimethyl-pyrazol-5-carbonsäure-methylester mit Methyliodid und Natriummethylat als Hilfsbase zu einem Gemisch der isomeren Trimethylpyrazolcarbonsäureester, die man erst "nach mehrfacher Rektifikation" in die Isomeren trennen konnte (J. Prakt. Chem. 126, 198 (1930)). Die Ethylierung von 3-Methylpyrazol-5-carbonsäure-ethylester mit Ethylbromid/Natrium in absolutem Alkohol ergibt ein Gemisch aus 1-Ethyl-3-methylpyrazol-5-carbonsäureethylester und 1-Ethyl-5-methyl-pyrazol-3-carbonsäureethylester im Verhältnis von ca. 1:3. D.h. das häufig gewünschte 1,3-Dialkylisomere wird im deutlichen Unterschuß gebildet und erst nach dreimaliger Destillation rein erhalten (Chem. Ber. 59, 603 (1926)).

Die andere, weithin verbreitete Methode, 1-Alkyl-pyrazol-5-carbonsäureester herzustellen besteht in der Umsetzung von 2,4-Diketocarbonsäureestern mit N-Alkylhydrazinen. Auch hier werden Isomerengemische erhalten, die in der Regel überwiegend das häufig unerwünschte Isomere enthalten, was dann ebenfalls ein aufwendiges Trennverfahren nötig macht. So ergibt die Umsetzung von 2,4-Dioxopentancarbonsäureethylester mit Methylhydrazin ein 1:1-Gemisch aus 1,5-Dimethylpyrazol-3-carbonsäureester und dem entsprechenden 2,5-Dimethylisomeren (Austr. J. Chem. 36, 135-147 (1983)). Andere Autoren berichten für diese Umsetzung noch ungünstigere Verhältnisse von 35:65 (Chem. Ber. 59, 1282 (1926)), die in vergleichenden Laborversuchen bestätigt wurden. Die gleichen Autoren haben mit analogen veretherten Enolen, z.B. mit O-Ethylacetonoxalester und Methylhydrazin, noch schlechtere Ergebnisse erzielt (Isomerenverhältnis 15:85). In der Regel werden bei diesen Umsetzungen entweder die freien Hydrazine mit dem Diketoester oder das Natriumsalz des Diketoesters (Enolat) und Hydrazinsalze eingesetzt, aus denen das Hydrazin mit Laugen wie Natriumhydroxid oder Soda in Freiheit gesetzt wird.

Die bei Herstellungsverfahren des Standes der Technik anfallenden Reaktionsgemische können nicht destillativ aufgearbeitet werden, weil sie Nebenprodukte enthalten, die eine destillative Trennung der beiden gebildeten Isomeren unmöglich machen.

Gemäß einem eigenen älteren Vorschlag werden 1-Alkyl-pyrazol-5-carbonsäureester hergestellt, indem man das Enolat eines 2,4-Diketocarbonsäureesters in Gegenwart eines Lösungsmittels, z.B. eines Alkohols, mit einem N-Alkylhydraziniumsalz umsetzt (deutsche Patentanmeldung 19 701 277.9). Dabei ist das N-Alkylhydraziniumsalz aus Alkylhydrazin mit einer Säure in Gegenwart von Alkohol herzustellen. Es müssen dabei Alkylhydrazine in reiner Form gehandhabt werden, was wegen deren Toxizität und explosiven Eigenschaften (siehe Römpp Lexikon Chemie-Version 1.3 (1997) - Methylhydrazin ist ein Rakententreibstoff) nur mit einem besonders großen sicherheitstechnischen Aufwand möglich ist.

Es besteht deshalb immer noch das Bedürfnis nach einem Verfahren zur selektiven Herstellung möglichst isomerenfreier 1-Alkyl-pyrazol-5-carbonsäureestern, das nicht so hohen sicherheitstechnischen Aufwand erfordert.

Es wurde nun ein Verfahren zur Herstellung von 1-Alkyl-pyrazol-5-carbonsäureestern der Formel (I) gefunden 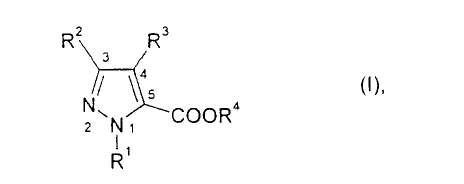 in der
- R¹ und R⁴: unabhängig voneinander jeweils für geradkettiges oder verzweigtes C₁-C₆-Alkyl, C₃-C₇-Cycloalkyl oder gegebenenfalls substituiertes C₇-C₁₂-- Aralkyl und
- R² und R³: unabhängig voneinander jeweils für Wasserstoff, geradkettiges oder verzweigtes C₁-C₆-Alkyl, C₃-C₇-Cycloalkyl oder gegebenenfalls substituiertes C₇-C₁₂-Aralkyl stehen,
das dadurch gekennzeichnet ist, daß man das Enolat eines 2,4-Diketocarbonsäureesters der Formel 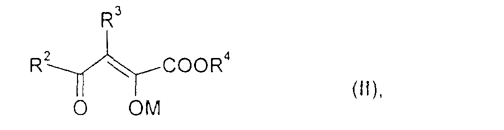 in der
- R², R³ und R⁴: die bei Formel (I) angegebene Bedeutung haben und
- M: für ein Äquivalent eines Metallatoms steht,
in Gegenwart von einem Lösungsmittel und von Wasser mit einem N-Alkylhydraziniumsalz der Formel (III) umsetzt

R¹-NH₂-NH₂^{⊕} R⁵COO^{θ} (III),

in der
- R¹: die bei Formel (I) angegebene Bedeutung hat und
- R⁵: zusammen mit dem COO^{θ}-Teil für das Anion einer organischen Säure steht.
C₇-C₁₂-Aralkyl und das daraus bevorzugte Benzyl sowie C₆-C₁₀-Aryl (später genannt) und das daraus bevorzugte Phenyl (später genannt) können beispielsweise jeweils bis zu zwei Substituenten aus der Gruppe der Halogenatome und der C₁-C₄-Alkylreste enthalten.

Als Diketocarbonsäureesterenolate der Formel (II) sind solche bevorzugt, bei denen die Reste R² und R³ unabhängig voneinander jeweils für Wasserstoff, geradkettiges oder verzweigtes C₁-C₄-Alkyl, C₃-C₆-Cycloalkyl oder gegebenenfalls substituiertes Benzyl stehen sowie solche, bei denen der Rest R⁴ für geradkettiges oder verzweigtes C₁-C₄-Alkyl steht.

Besonders bevorzugt sind 2,4-Diketocarbonsäureesterenolate der Formel (II), bei denen R² und R⁴ jeweils für C₁-C₄-Alkyl und R³ für H stehen.

M in der Formel (II) steht bevorzugt für ein einwertiges Metallatom oder ein Äquivalent eines zweiwertigen Metallatoms. Beispiele sind Lithium, Natrium, Kalium, Calcium und Magnesium. Besonders bevorzugt sind Natrium, Lithium und Magnesium.

Von den N-Alkylhydraziniumsalzen der Formel (III) sind solche bevorzugt, bei denen R¹ für geradkettiges oder verzweigtes C₁-C₄-Alkyl oder gegebenenfalls substituiertes Benzyl und R⁵ für geradkettiges oder verzweigtes C₁-C₆-Alkyl, C₃-C₇-Cycloalkyl, C₇-C₁₂-Aralkyl oder C₆-C₁₀-Aryl stehen, die gegebenenfalls wie oben angegeben substituiert sein können.

R⁵ kann zusammen mit dem COO^{θ}-Teil auch für ein Anion einer mehrbasischen organischen Säure stehen. Beispiele sind Anionen von Oxal-, Malon-, Bernstein-, Glutar-, Adipin-, Malein-, Fumar-, Äpfel-, Wein- und Zitronensäure, wobei es sich jeweils um Mono- oder Polyanionen handeln kann. Solche Anionen können einen oder mehrere COO^{θ}-Teile und gegebenenfalls zusätzlich auch COOH-Reste enthalten. Im Falle von Anionen von mehrbasischen organischen Säuren kann das Alkylhydrazin im Verhältnis zur Säure in äquimolaren Mengen oder in Mengen, die der Anzahl der Säuregruppen entspricht, eingesetzt werden.

Die Diketoesterenolate der Formel (II) können z.B. aus den entsprechenden, reinen, isolierten 2,4-Diketoestern durch Zugabe eines Alkoholats hergestellt werden. Als Alkoholate kommen z.B. solche in Frage, die der Formel (IV) entsprechen

M(OR⁶)ₙ (IV),

in der
- M: die bei Formel (II) angegebene Bedeutung hat,
- R⁶: für C₁-C₄-Alkyl steht und
- n: der Wertigkeit von M entspricht.

Die Herstellung der Diketoesterenolate der Formel (II) kann in einem Lösungsmittel, beispielsweise in einem C₁-C₄-Alkohol erfolgen.

Diketoesterenolate der Formel (II) können auch nach der üblichen Methode durch Kondensation eines Methylalkylketons der Formel (V) 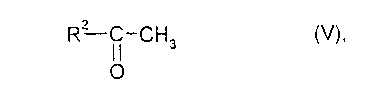 in der
- R²: die bei Formel (I) angegebene Bedeutung hat,
mit einem Oxalester der Formel

R⁴OOC-COOR⁴ (VI)

in der
- R⁴: die bei Formel (I) angegebene Bedeutung hat,
hergestellt werden. Auch hier arbeitet man in Gegenwart eines Alkoholats, beispielsweise eines Alkoholats der Formel (IV) und eines Lösungsmittels. Die dabei erhaltene rohe Reaktionsmischung kann direkt in die Umsetzung mit dem N-Alkylhydraziniumsalz der Formel (III) eingesetzt werden.

Als Lösungsmittel für die Umsetzung der Methylalkylketone der Formel (V) mit Oxalestern der Formel (VI) und Weiterreaktion mit den N-Alkylhydraziniumsalzen der Formel (III) kommen beispielsweise Alkohole wie Methanol, Ethanol, Isopropanol, n-Propanol und n-, i-, s- und t-Butanol in Frage. Das Alkoholat der Formel (IV) kann durch Auflösen eines Alkali- oder Erdalkalimetalls M in dem Alkohol hergestellt werden, der dem Alkoholat der Formel (IV) entspricht.

Wasser wird vorteilhafterweise zusammen mit dem Hydraziniumsalz der Formel (III) oder anschließend daran in das Reaktionsgemisch eingebracht.

Das Hydraziniumsalz der Formel (III) kann z.B. durch Mischen eines Alkylhydrazins der Formel (VII)

R¹-NH-NH₂ (VII),

in der
- R¹: die bei Formel (I) angegebene Bedeutung hat,
mit einer Carbonsäure der Formel (VIII) hergestellt werden

R⁵-COOH (VIII),

in der
- R⁵: die bei Formel (III) angegebene Bedeutung hat.

Man kann dabei gegebenenfalls unter Zusatz eines Lösungsmittels, z.B. eines Alkohols arbeiten.

Bevorzugt arbeitet man so, daß man eine Lösung von Hydraziniumsalzen der Formel (III) einsetzt, die man erhalten hat, indem man eine wäßrige Lösung eines Alkylhydrazins (z.B. der Formel (VII)) mit einer Carbonsäure (z.B. der Formel (VIII)) umsetzt. Man kann auf diese Weise den Umgang mit reinen Alkylhydrazinen vermeiden und den für den Umgang mit reinen Alkylhydrazinen erforderlichen großen sicherheitstechnischen Aufwand einsparen. So kann die Wirtschaftlichkeit der Herstellung von 1-Alkyl-pyrazol-5-carbonsäureester beträchtlich erhöht werden.

Zur Herstellung von Verbindungen der Formel (I) werden das Enolat der Formel (II) und das N-Alkylhydraziniumsalz der Formel (III), beide vorteilhafterweise in Form der bei ihrer Herstellung anfallenden alkoholischen bzw. wäßrigen Lösung, zusammengefügt. Eventuell zur Ausfällung neigende Anteile der Enolate oder der N-Alkylhydraziniumsalze können durch Erwärmen und/oder Zugabe von weiterem Lösungsmittel und/oder Wasser wieder in Lösung gebracht oder gehalten werden. Zum Beispiel kann eine alkoholische Enolatlösung zu einer vorgelegten wäßrigen N-Alkylhydraziniumsalzlösung oder umgekehrt eine wäßrige N-Alkylhydraziniumsalzlösung zu einer vorgelegten alkoholischen Enolatlösung zugegeben werden.

Die insgesamt eingesetzten Lösungsmittel- und Wassermengen werden im allgemeinen so gewählt, daß rührbare Suspensionen oder Lösungen vorliegen. Die Gesamtmenge Lösungsmittel plus Wasser pro Mol Reaktionsansatz kann z.B. zwischen 100 und 2000 ml liegen. Bevorzugt beträgt diese Menge 200 bis 1000 ml, besonders bevorzugt 250 bis 500 ml. Von der Gesamtmenge Lösungsmittel plus Wasser können beispielsweise 10 bis 60 Gew.-%, vorzugsweise 15 bis 40 Gew.-% Wasser sein.

Das Molverhältnis für die Umsetzung der Diketoesterenolate der Formel (II) mit den N-Alkylhydraziniumsalzen der Formel (III) kann in weiten Grenzen schwanken, beispielsweise von 5:1 bis 1:5. In einer bevorzugten Ausführungsform werden etwa äquimolare Mengen Diketoesterenolat der Formel (II) und N-Alkylhydraziniumsalz der Formel (III) zur Reaktion gebracht, beispielsweise 0,9 bis 1,1 Mole Diketoesterenolat der Formel (II) pro Mol N-Alkylhydraziniumsalz der Formel (III).

Die Alkoholatmenge zur Herstellung des Diketoesterenolats kann ebenfalls in weiten Grenzen schwanken. Vorzugsweise ist sie zumindest äquimolar.

Wird der Diketoester in einem vorgeschalteten Schritt wie oben beschrieben aus einem Keton der Formel (V) und einem Oxalsäureester der Formel (VI) hergestellt, so können auch deren Molverhältnisse variieren. Bevorzugt arbeitet man mit einem leichten Unterschuß, z.B. je mit einem Unterschuß von 1 bis 10 Mol-% an dem Keton der Formel (V) bezogen auf den Oxalester der Formel (VI) und das Alkoholat der Formel (IV). Die beiden letzteren werden bevorzugt in etwa äquimolaren Mengen zueinander eingesetzt. Die Molverhältnisse betragen dann beispielsweise 0,9 bis 0,99 Mol Keton der Formel (V) zu 0,9 bis 1,1 Mol Oxalsäureester der Formel (VI) zu 0,9 bis 1,1 Mol Alkoholat der Formel (IV).

Bei der Herstellung des N-Alkylhydraziniumsalzes der Formel (III) aus einem Alkylhydrazin der Formel (VII), vorzugsweise einer wäßrigen Lösung eines Alkylhydrazins der Formel (III), und einer Carbonsäure der Formel (VIII) kann das Molverhältnis der beiden Einsatzstoffe ebenfalls in weiten Grenzen schwanken. Bevorzugt ist eine Menge von 1 bis 1,5 Mol Säure pro Mol Alkylhydrazin. Besonders bevorzugt ist der Einsatz äquimolarer Mengen Alkylhydrazin und Carbonsäure.

Ein Überschuß an Carbonsäure der Formel (VIII) ist vorteilhaft, wenn Alkoholat der Formel (IV) im Überschuß vorliegt. Dann kann dieser Alkoholatüberschuß mit dem Säureüberschuß neutralisiert werden.

Die Reaktionstemperaturen für die Umsetzung der Reaktionspartner der Formel (II) und (III) können z.B. zwischen -20 und +100°C liegen, bevorzugt zwischen 0 und 80°C, besonders bevorzugt zwischen 20 und 50°C. Dies gilt auch für die Temperaturen während der Nachrührzeiten.

Die Reaktionszeiten (Zusammengeben der Reaktionspartner + Nachrührzeit) können z.B. zwischen 0,5 und 12 Stunden, bevorzugt zwischen 1 und 8 Stunden, besonders bevorzugt zwischen 2 und 5 Stunden betragen. Für das erfindungsgemäße Verfahren werden bevorzugt folgende Verbindungen der Formel (II) eingesetzt: 2,4-Diketopentancarbonsäureethylester als Natrium-, Lithium-, Kalium- oder Magnesiumenolat, 2,4-Diketohexancarbonsäureethylester, 2,4-Diketoheptancarbonsäureethylester, 2,4-Diketooctancarbonsäureethylester und 2,4-Diketo-3-ethylpentancarbonsäureethylester (jeweils in Form ihrer Natrium-, Lithium-, Magnesium- oder Kaliumenolatsalze) und Methyl-, n-Propyl-, i-Propyl- und n-, i-, s- und t-Butylester der zuvor genannten Diketocarbonsäuren ebenfalls in Form der genannten Enolatsalze.

N-Alkylhydraziniumsalze der Formel (III) sind bevorzugt Methylhydraziniumformiat, -acetat, -propionat, -butyrat, -isobutyrat, -benzoat, -oxalat und -succinat, N-Propylhydraziniumforminat, -acetat, -propionat, -butyrat, -isobutyrat und -benzoat, Ethylhydraziniumformiat, -acetat, -propionat, -butyrat, -ibutyrat, -benzoat, -oxalat und -succinat sowie die Hydraziniumsalze von i-Propyl-, n-Butyl-, t-Butyl-, Benzyl- und n-Pentylhydrazin mit den vorgenannten Carbonsäureanionen.

Eine allgemeine Ausführungsform des erfindungsgemäßen Verfahrens, beispielhaft an der Umsetzung von 2,4-Diketoheptancarbonsäureethylester-Natrium-Salz mit Methylhydraziniumformiat erläutert, ist wie folgt:

Aus 2-Pentanon und Oxalester wird mit Natriumethylat als Hilfsbase in Ethanol das Natriumsalz des 2,4-Diketoheptancarbonsäureethylesters analog bekannter Vorschriften hergestellt (siehe z.B. Organikum, 19. Auflg., S. 490 (1993)). Diese Lösung wird, um Ausfallen des Enolates zu verhindern, auf 50°C gehalten und innerhalb von 1 Stunde zu einer zuvor hergestellten Lösung von Methylhydraziniumformiat in Wasser gegeben (Methylhydrazin in Wasser vorlegen, Ameisensaüre zutropfen). Man rührt eine angemessene Zeit nach, destilliert das überschüssige Ethanol ab und versetzt mit Toluol und gegebenenfalls weiterem Wasser. Zur Verbesserung der Trennung der Phasen kann man gegebenenfalls ein geeignetes Tensid, z.B. ein Alkansulfonat, zufügen und/oder die Ionenkonzentration in der wäßrigen Phase erhöhen, z.B. durch Zusatz eines Salzes. Die Toluolphase wird abgetrennt, die wäßrige Phase noch 2 mal mit Toluol extrahiert und nach Vereinigung der organischen Phasen diese mit Wasser reextrahiert. Das Wasser kann gegebenenfalls eine Säure und/oder ein Salz enthalten. Die toluolische Lösung des Rohpyrazols wird durch Destillation des Lösungsmittels eingeengt und der Rückstand im Vakuum fraktioniert destilliert. Die beiden isomeren Pyrazole lassen sich ohne großen Trennaufwand in reiner Form isolieren.

Es ist ausgesprochen überraschend, daß auch in Gegenwart von Wasser der Einsatz der N-Alkylhydraziniumsalze zur Herstellung von N-Alkylpyrazolen die Regioselektivität günstig beeinflußt. Nach dem Stand der Technik wird beim Einsatz freier Alkylhydrazine (ob als solche eingesetzt oder durch Alkalilaugen aus ihren Salzen freigesetzt ist unerheblich) das unerwünschte Isomere mit dem Alkylrest am von der Carboxylgruppe entfernteren Stickstoff bevorzugt gebildet, während der Einsatz von N-Alkylhydraziniumsalzen der Formel (III) bevorzugt das gewünschte Isomere (I) liefert, bei dem die Alkylgruppe an dem Stickstoff steht, der der Carbonylgruppe im Pyrazol am nächsten steht. Überraschenderweise kann man durch die erfindungsgemäße Gegenwart von Wasser den Aufwand für die Handhabung von reinem Alkylhydrazin vermeiden und trotzdem das gewünschte Isomere in hohen Anteilen erhalten.

1-Alkyl-pyrazol-5-carbonsäureester der Formel (I) sind wertvolle Zwischenprodukte zur Herstellung von pharmazeutischen Wirkstoffen mit gefäßverändernder und/oder krampflösender Wirkung (siehe EP-OS 463 756, EP-OS 526 004 und DE-OS 19 27 429) sowie zur Herstellung von Pestiziden mit insektizider und akarizider Wirkung (siehe JP-OS 89-114 466).

### Beispiel

### 1-Methyl-3-n-propylpyrazol-5-carbonsäure-ethylester

In einem 1 l-Vierhalskolben wurden 146,1 g Oxalsäurediethylester vorgelegt und dazu 86,1 g Pentanon-2 und 3 g Ethanol gegeben. Unter Rühren wurden bei 25 bis 40°C 340,3 g einer 20 gew.-%igen Natriumethylatlösung innerhalb von einer Stunde zudosiert, anschließend wurde mit 3 g Ethanol nachgespült. Das Reaktionsgemisch wurde 1 Stunde bei 50°C und anschließend 1 Stunde bei 80°C gerührt und nachfolgend auf 50°C abgekühlt. In einem weiteren Kolben wurden während der Nachrührzeit 126,7 g einer 40 gew.-%igen wäßrigen Methylhydrazin-Lösung vorgelegt, hierzu 66 g Essigsäure innerhalb von einer Stunde bei 5 bis 30°C zugetropft und anschließend auf 0°C gekühlt. Zu diesem Gemisch wurde die oben hergestellte, auf 50°C gehaltene 2,4-Diketoheptansäurcethylesterenolat-Lösung innerhalb von 2 Stunden zugetropft, so daß die Temperatur des Reaktionsgemisches zwischen 0 und 10°C und die Temperatur der 2,4-Diketoheptansäureethylesterenolat-Lösung bei ca. 50°C gehalten wurde. Es wurde mit 3 g Ethanol nachgespült. Anschließend wurde Lösungsmittel abdestilliert, bis eine Sumpftemperatur von 88°C erreicht war. Der Sumpf wurde abgekühlt und unter Rühren mit 300 ml Toluol, 500 ml Wasser und 45 g Mersolat® H 30 und 20 g Natriumchlorid versetzt. Die wäßrige Phase wurden abgetrennt und mit 100 ml Toluol gewaschen. Die organischen Phasen wurden vereinigt, mit 210 g 10 gew.-%iger Schwefelsäure und 200 ml 10 gew.-%iger Natriumchloridlösung gewaschen und anschließend bei 60 mbar eingeengt, bis eine Sumpftemperatur von 70°C erreicht wurde. Es wurde 211 g eines braunen Öles erhalten, dessen Zusammensetzung 54,2 gew.-% an 1-Methyl-3-n-propylpyrazol-5-carbonsäure-ethylester und 19,5 Gew.-% des "Fehlisomeren" 1-Methyl-5-n-propylpyrazol-3-carbonsäure-ethylester enthielt, was einer Rohausbeute von 58,3% der gewünschten Verbindung entspricht.

## Patentansprüche

1. Verfahren zur Herstellung von 1-Alkyl-pyrazol-5-carbonsäureestern der Formel (I) 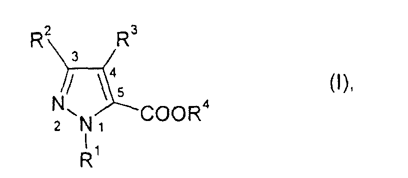 in der
R¹ und R⁴ unabhängig voneinander jeweils für geradkettiges oder verzweigtes C₁-C₆-Alkyl, C₃-C₇-Cycloalkyl oder gegebenenfalls substituiertes C₇-C₁₂-Aralkyl und
R² und R³ unabhängig voneinander jeweils für Wasserstoff, geradkettiges oder verzweigtes C₁-C₆-Alkyl, C₃-C₇-Cycloalkyl oder gegebenenfalls substituiertes C₇-C₁₂-Aralkyl stehen,
**dadurch gekennzeichnet, daß** man das Enolat eines 2,4-Diketocarbonsäureesters der Formel 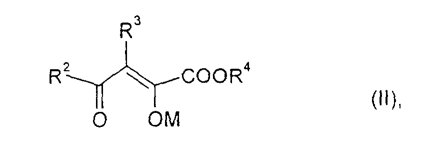 in der
R², R³ und R⁴ die bei Formel (I) angegebene Bedeutung haben und
M für ein Äquivalent eines Metallatoms steht,
in Gegenwart von einem Lösungsmittel und von Wasser mit einem N-Alkylhydraziniumsalz der Formel (III) umsetzt
R¹-NH₂-NH₂^{⊕} R⁵COO^{θ} (III),
in der
R¹ die bei Formel (I) angegebene Bedeutung hat und
R⁵ zusammen mit dem COO^{θ}-Teil für das Anion einer organischen Säure steht.

2. Verfahren nach Anspruch 1, **dadurch gekennzeichnet, daß** es sich bei den Diketocarbonsäureesterenolaten der Formel (II) um solche handelt, bei denen die Reste R² und R³ unabhängig voneinander jeweils für Wasserstoff, geradkettiges oder verzweigtes C₁-C₄-Alkyl, C₃-C₆-Cycloalkyl oder gegebenenfalls substituiertes Benzyl stehen sowie solche, bei denen der Rest R⁴ für geradkettiges oder verzweigtes C₁-C₄-Alkyl und bei denen M für Lithium, Natrium, Kalium, Calcium oder Magnesium steht.

3. Verfahren nach Ansprüchen 1 bis 2, **dadurch gekennzeichnet, daß** es sich bei den N-Alkylhydraziniumsalzen der Formel (III) um solche handelt, bei denen R¹ für geradkettiges oder verzweigtes C₁-C₄-Alkyl oder gegebenenfalls substituiertes Benzyl und R⁵ für geradkettiges oder verzweigtes C₁-C₆-Alkyl, C₃-C₇-Cycloalkyl, C₇-C₁₂-Aralkyl oder C₆-C₁₀-Aryl stehen, die gegebenenfalls substituiert sein können.

4. Verfahren nach Ansprüchen 1 bis 3, **dadurch gekennzeichnet, daß** in Formel (III) R⁵ zusammen mit dem COO^{θ}-Teil für ein Anion einer mehrbasischen organischen Säure wie Oxal-, Malon-, Bernstein-, Glutar-, Adipin-, Malein-, Fumar-, Äpfel-, Wein- oder Zitronensäure steht.

5. Verfahren nach Ansprüchen 1 bis 4, **dadurch gekennzeichnet, daß** man Wasser zusammen mit den N-Alkylhydraziniumsalz der Formel (III) oder anschließend daran in das Reaktionsgemisch einbringt.

6. Verfahren nach Ansprüchen 1 bis 5, **dadurch gekennzeichnet, daß** man eine Lösung eines N-Alkylhydraziniumsalzes der Formel (III) einsetzt, die durch Umsetzung einer wäßrigen Lösung eines Alkylhydrazins mit einer Carbonsäure erhalten wurde.

7. Verfahren nach Ansprüchen 1 bis 6, **dadurch gekennzeichnet, daß** man die Diketocsterenolate der Formel (II) aus den entsprechenden, reinen, isolierten 2,4-Diketoestern durch Zugabe eines Alkoholats in Gegenwart eines Lösungsmittels herstellt.

8. Verfahren nach Ansprüchen 1 bis 7, **dadurch gekennzeichnet, daß** pro Mol Reaktionsansatz 100 bis 2 000 ml Lösungsmittel plus Wasser vorliegen.

9. Verfahren nach Ansprüchen 1 bis 8, **dadurch gekennzeichnet, daß** das Molverhältnis für die Umsetzung der Diketoesterenolate der Formel (II) mit den N-Alkylhydraziniumsalzen der Formel (III) zwischen 5:1 und 1:5 liegt und für die Herstellung des Dikctoesterenolats die Alkoholatmenge zumindest äquimolar ist, wobei dann die Molverhältnisse 0,9 bis 0.99 Mol Keton der Formel (V) zu 0,9 bis 1,1 Mol Oxalsäureester der Formel (VI) zu 0,9 bis 1,1 Mol Alkoholat der Formel (IV) betragen.

10. Verfahren nach Ansprüchen 1 bis 9, **dadurch gekennzeichnet, daß** die Reaktionstemperatur für die Umsetzung der Reaktionspartner der Formeln (II) und (III) zwischen -20 und +100°C und die Reaktionszeiten zwischen 0,5 und 12 Stunden liegen.

## Claims

1. Process for preparing 1-alkyl-pyrazole-5-carboxylic esters of the formula (I) 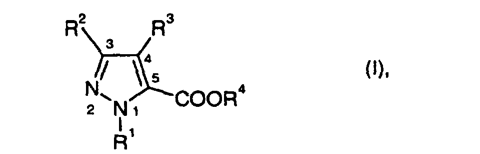 in which
R¹ and R⁴ independently of one another each represent straight-chain or branched C₁-C₆-alkyl, C₃-C₇-cycloalkyl or optionally substituted C₇-C₁₂-aralkyl and
R² and R³ independently of one another each represent hydrogen, straight-chain or branched C₁-C₆-alkyl, C₃-C₇-cycloalkyl or optionally substituted C₇-C₁₂-aralkyl,
**characterized in that** it comprises reacting the enolate of a 2,4-diketocarboxylic ester of the formula 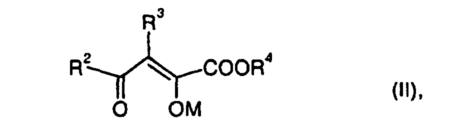 in which
R2, R3 and R4 are each as defined in formula (I) and
M represents the equivalent of a metal atom,
in the presence of a solvent and of water with an N-alkylhydrazinium salt of the formula (III)
R¹-NH₂-NH₂^{⊕} R⁵COO^{θ} (III),
in which
R1 is as defined in formula (I) and
R5 together with the COO^{θ} moiety represents the anion of an organic acid.

2. Process according to Claim 1, charcaterized in that the diketocarboxylic ester enolates of the formula (II) are those where the radicals R² and R³ independently of one another each represent hydrogen, straight-chain or branched C₁-C₄-alkyl, C₃-C₆-cycloalkyl or optionally substituted benzyl and also those where the radical R⁴ represents straight-chain or branched C₁-C₄-alkyl and where M represents lithium, sodium, potassium, calcium or magnesium.

3. Process according to Claims 1 and 2, **characterized in that** the N-alkylhydrazinium salts of the formula (III) are those where R¹ represents straight-chain or branched C₁-C₄-alkyl or optionally substituted benzyl and R⁵ represents straight-chain or branched C₁-C₆-alkyl, C₃-C₇-cycloalkyl, C₇-C₁₂-aralkyl or C₆-C₁₀-aryl, which may optionally be substituted.

4. Process according to Claims 1 to 3, **characterized in that** in formula (III) R⁵ together with the COO^{θ} moiety represents an anion of a polybasic organic acid such as oxalic, malonic, succinic, glutaric, adipic, maleic, fumaric, malic, tartaric or citric acid.

5. Process according to Claims 1 to 5, **characterized in that** water is introduced into the reaction mixture together with the N-alkylhydrazinium salt of the formula (III), or subsequently thereto.

6. Process according to Claims 1 to 5, **characterized in that** a solution of an N-alkylhydrazinium salt of the formula (III) is used which was obtained by reaction of an aqueous solution of an alkylhydrazine with a carboxylic acid.

7. Process according to Claims 1 to 6, **characterized in that** the diketo ester enolates of formula (II) are prepared from the corresponding pure isolated 2,4-diketo esters by addition of an alkoxide in the presence of a solvent.

8. Process according to Claims 1 to 7, **characterized in that** 100 to 2000 ml of solvent and water are present per mole of reaction mixture.

9. Process according to Claims 1 to 8, **characterized in that** the molar ratio for the reaction of the diketo ester enolates of the formula (II) with the N-alkylhydrazinium salts of the formula (III) is beween 5:1 and 1:5 and the amount of alcohol for the preparation of the diketo ester enolate is at least equimolar, and where the molar ratios are then from 0.9 to 0.99 mol of ketone of the formula (V) to from 0.9 to 1.1 mol of oxalic ester of the formula (VI) to from 0.9 to 1.1 mol of alkoxide of the formula (IV).

10. Process according to Claims 1 to 9, **characterized in that** the reaction temperature for the reaction of the reaction components of the formulae (II) and (III) is between -20 and +100°C and the reaction times are between 0.5 and 12 hours.

## Revendications

1. Procédé pour la préparation des esters 1-alkylpyrazole-5-carboxyliques de formule (I) 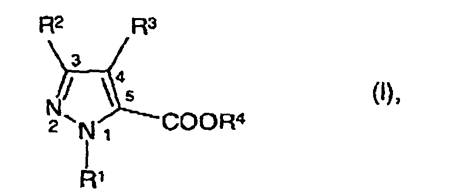 dans laquelle
R¹ et R⁴ représentent chacun, indépendamment l'un de l'autre, un groupe alkyle à chaîne droite ou ramifiée en C₁-C₆, cycloalkyle en C₃-C₇ ou aralkyle en C₇-C₁₂ éventuellement substitué et
R² et R³ représentent chacun, indépendamment l'un de l'autre, l'hydrogène, un groupe alkyle à chaîne droite ou ramifiée en C₁-C₆, cycloalkyle en C₃-C₇ ou aralkyle en C₇-C₁₂ éventuellement substitué,
**caractérisé en ce que** l'on fait réagir l'énolate d'un ester 2,4-dicétocarboxylique de formule 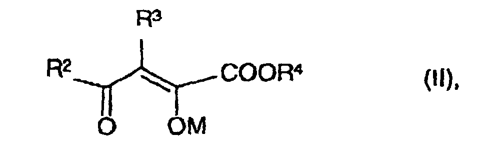 dans laquelle
R², R³ et R⁴ ont les significations indiquées en référence à la formule (I) et
M représente un équivalent d'un atome métallique,
en presence d'un solvant et d'eau, avec un sel de N-alkylhydrazinium de formule (III)
R¹-NH₂-NH₂^{⊕} R⁵COO^{θ} (III),
dans laquelle
R¹ a les significations indiquées en référence à la formule (I) et
R⁵ forme, avec la partie COO^{θ}, l'anion d'un acide organique.

2. Procédé selon la revendication 1, **caractérisé en ce que** les énolates d'esters dicétocarboxyliques de formule (II) sont les composés pour lesquels R² et R³ représentent chacun, indépendamment l'un de l'autre, l'hydrogène, un groupe alkyle à chaîne droite ou ramifiée en C₁-C₄, cycloalkyle en C₃-C₆ ou benzyle éventuellement substitué ou ceux pour lesquels R⁴ représente un groupe alkyle à chaîne droite ou ramifiée en C₁-C₄ et M le lithium, le sodium, le potassium, le calcium ou le magnésium.

3. Procédé selon les revendications 1 à 2, **caractérisé en ce que** les sels de N-alkylhydrazinium de formule (III) sont les composés pour lesquels R¹ représente un groupe alkyle à chaîne droite ou ramifiée en C₁-C₄ ou benzyle éventuellement substitué et R⁵ représente un groupe alkyle à chaîne droite ou ramifiée en C₁-C₆, cycloalkyle en C₃-C₇, aralkyle en C₇-C₁₂ ou aryle en C₆-C₁₀, éventuellement substitués.

4. Procédé selon les revendications 1 à 3, **caractérisé en ce que**, dans la formule (III), R⁵ forme avec la partie COO^{θ} un anion d'un acide organique polybasique tel que l'acide oxalique, l'acide malonique, l'acide succinique, l'acide glutarique, l'acide adipique, l'acide maléique, l'acide fumarique, l'acide malique, l'acide tartrique ou l'acide citrique.

5. Procédé selon les revendications 1 à 4, **caractérisé en ce que** l'on introduit l'eau dans le mélange de réaction avec le sel de N-alkylhydrazinium de formule (III) ou ultérieurement.

6. Procédé selon les revendications 1 à 5, **caractérisé en ce que** l'on met en oeuvre une solution d'un sel de N-alkylhydrazinium de formule (III) qui a été obtenue par réaction d'une solution aqueuse d'une alkylhydrazine avec un acide carboxylique.

7. Procédé selon les revendications 1 à 6, **caractérisé en ce que** l'on prépare les énolates de dicétoesters de formule (II) à partir des 2,4-dicétoesters correspondants isolés et à l'état pur par addition d'un alcoolate en présence d'un solvant.

8. Procédé suivant les revendications 1 à 7, **caractérisé en ce que** l'on utilise de 100 à 2 000 ml de solvant plus eau par mole du mélange de réaction.

9. Procédé selon les revendications 1 à 8, **caractérisé en ce que**, pour la réaction des énolates de dicétoester de formule (II) avec les sels de N-alkylhydrazinium de formule(III), le rapport molaire va de 5:1 à 1:5 et pour la préparation de l'énolate de dicétoester, la quantité d'alcool est au moins équimoléculaire, de sorte que les proportions molaires sont de 0,9 à 0,99 mol de la cétone de formule (V) pour 0,9 à 1,1 mol de l'ester oxalique de formule (VI) et 0,9 à 1,1 mol de l'alcoolate de formule (IV).

10. Procédé selon les revendications 1 à 9, **caractérisé en ce que** la température, pour la réaction entre les composants de formule (II) et (III), va de -20 à +100°C et la durée de réaction est de 0,5 à 12 h.
